# EUROPEAN PATENT APPLICATION

(11) **EP 4 801 231 A1**
(43) Date of publication of application: **02.09.2026**
(21) Application number: 24882478.1
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **PHOTOELECTRIC CONVERSION ELEMENT AND PHOTOELECTRIC CONVERSION DEVICE**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024086015; 23.10.2024 JP 2024186450
(71) Applicant: Canon Kabushiki Kaisha, Tokyo 146-8501 (JP)
(72) Inventor: OHSAWA, Tatsuya, Tokyo 146-8501 (JP); NAKAMURA, Nobuhiro, Tokyo 146-8501 (JP); SEKIDO, Kunihiko, Tokyo 146-8501 (JP); NISHIDA, Tsutomu, Tokyo 146-8501 (JP)
(74) Representative: TBK
(86) International application number: PCT/JP2024/038052
(87) International publication number: WO 2025/089369

(57) **Abstract**

Provided is a photoelectric conversion element in which a defect of a crystal having a perovskite structure is protected, and leakage resistance property and conversion efficiency are improved.

The photoelectric conversion element includes: a first electrode; a second electrode; and a photoelectric conversion layer arranged between the first electrode and the second electrode, the photoelectric conversion layer containing a crystal having a perovskite structure. The photoelectric conversion element includes, between the photoelectric conversion layer and the first electrode, a charge-transporting layer containing: a cyclic conjugated compound in which a plurality of pyrrole rings are bonded by conjugated bonds; and an aliphatic resin having at least one kind of functional group selected from the group consisting of: a hydroxy group; and a carboxy group.

## Description

### [Technical Field]

The present invention relates to a photoelectric conversion element and a photoelectric conversion apparatus.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage utilizing a photovoltaic effect in which light energy is absorbed from sunlight to generate an electron and a hole.

Recently, an n-p diode-type silicon (Si) single crystal-based solar cell having a light energy conversion efficiency of more than 20% has been widely known, and has been actually used in solar power generation. However, the solar cell requires a high temperature treatment step and the price of a material itself is high, and hence there is a problem in that the cost per unit electric power is high. In addition, there is a problem with a supply property in terms of a silicon resource.

Meanwhile, a solar cell using an organic material (hereinafter also referred to as "organic solar cell") does not require the high temperature treatment step, and is a sheet-shaped substrate, which can be produced in a so-called roll to roll system. Accordingly, a cost reduction is expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion property.

For example, in Patent Literature 1, there is a description that the peeling of a hole-transporting layer from an anode is suppressed by mixing an insulating polymer and a hole-transporting material thereinto, and hence conversion efficiency and durability are improved. In Non Patent Literature 1, there is a description of an improvement in conversion efficiency because of the suppression of shunt leakage by incorporating polymethyl methacrylate (PMMA) serving as a leakage prevention layer into an upper layer of perovskite. In Non Patent Literature 2, there is a description that conversion efficiency is improved by mixing copper phthalocyanine and a conductive polymer into a hole-transporting layer.

### [Citation List]

### [Patent Literature]

PTL 1: Japanese Patent Laid-Open No. 2018-170382

### [Non Patent Literature]

NPL 1: F. Wang, et al, J. Phys. Chem. C, 2017, 121, 1562
NPL 2: Q. Hu, et al, Sol. RRL, 2019, 3, 1800264

### [Summary of Invention]

### [Technical Problem]

According to investigations made by the inventors of the present invention, in each of the photoelectric conversion elements described in Patent Literature 1, and Non Patent Literatures 1 and 2, there has been a problem in realizing a further improvement in conversion efficiency while maintaining the suppression of shunt leakage.

Accordingly, the present invention is directed to providing a photoelectric conversion element in which a defect of a crystal having a perovskite structure is protected, and leakage resistance property and conversion efficiency are improved. In addition, the present invention is directed to providing a photoelectric conversion device in which leakage resistance property and conversion efficiency are improved.

### [Solution to Problem]

The above-mentioned provision is achieved by the following present invention. That is, a photoelectric conversion element according to the present invention is a photoelectric conversion element including: a first electrode; a second electrode; and a photoelectric conversion layer arranged between the first electrode and the second electrode, the photoelectric conversion layer containing a crystal having a perovskite structure, wherein the photoelectric conversion element further includes, between the photoelectric conversion layer and the first electrode, a charge-transporting layer containing: a cyclic conjugated compound in which a plurality of pyrrole rings are bonded by conjugated bonds; and an aliphatic resin having at least one kind of functional group selected from the group consisting of: a hydroxy group; and a carboxy group.

### [Advantageous Effects of Invention]

According to the present invention, the photoelectric conversion element in which leakage resistance property and conversion efficiency are improved can be provided.

### [Brief Description of Drawings]

[Fig. 1]

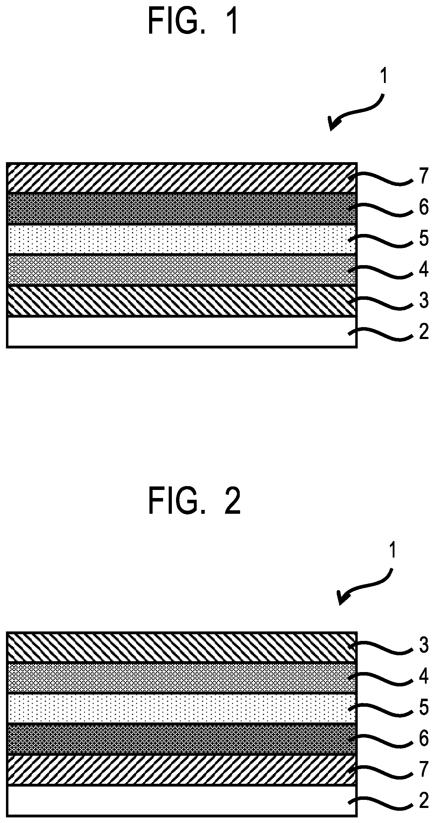

   Fig. 1 is a schematic sectional view in the thickness direction of a photoelectric conversion element according to a first embodiment of the present invention.
[Fig. 2]
   Fig. 2 is a schematic sectional view in the thickness direction of a photoelectric conversion element according to a second embodiment of the present invention.
[Fig. 3]

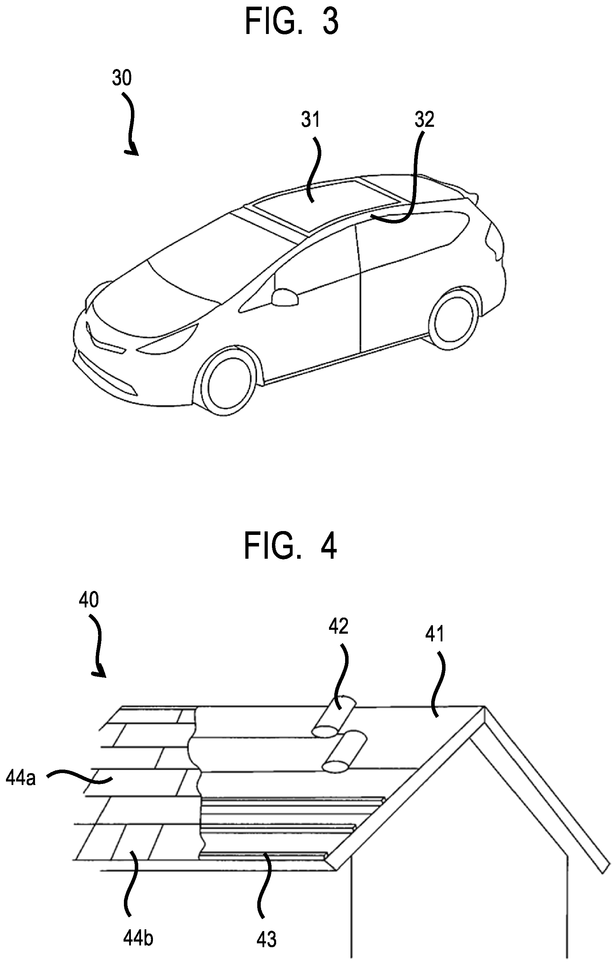

   Fig. 3 is a perspective view for schematically illustrating an embodiment of a moving body including the photoelectric conversion element of the present invention.
[Fig. 4]
   Fig. 4 is a perspective view for schematically illustrating an embodiment of a building material including the photoelectric conversion element of the present invention.

### [Description of Embodiments]

A photoelectric conversion element of the present invention includes a first electrode, a second electrode, and a photoelectric conversion layer arranged between the first electrode and the second electrode, the photoelectric conversion layer containing a crystal having a perovskite structure, and includes, between the photoelectric conversion layer and the first electrode, a charge-transporting layer containing: a cyclic conjugated compound in which a plurality of pyrrole rings are covalently bonded; and an aliphatic resin having at least one kind of functional group selected from the group consisting of: a hydroxy group; and a carboxy group.

As a result of investigations, the inventors of the present invention have found that, when the above-mentioned charge-transporting layer is included, a photoelectric conversion element excellent in leakage resistance property and conversion efficiency is obtained. The reason why the photoelectric conversion element having high stability can be obtained in the present invention is not clear in detail, but is conceived as described below.

High hole-transporting ability is exhibited by forming a film through use of the cyclic conjugated compound in which the plurality of pyrrole rings are covalently bonded as a charge-transporting material. Further, in the related-art investigations made by the inventors, it is presumed that, when the photoelectric conversion layer contains the crystal having a perovskite structure, submicron unevenness occurs on its surface, and hence interface bonding is stabilized and high photoelectric conversion efficiency can be obtained by filling a recess of such unevenness with a pigment particle formed of a phthalocyanine compound. However, it has been found that leakage due to a crystal defect or deterioration of the crystal having a perovskite structure cannot be suppressed by the filling of the pigment including the phthalocyanine compound in some cases. Further, according to investigations made by the inventors, it has been found that there is room for further improvement in leakage resistance property in the photoelectric conversion element described in Non Patent Literature 1 because film formation with an extremely thin film of an insulating resin is required from the viewpoint of conductivity, and hence it is difficult to completely cover a crystal having a perovskite structure having a large defect site.

Accordingly, in the present invention, improvements in leakage resistance property and conversion efficiency are enabled by forming a charge-transporting layer containing: a cyclic conjugated compound in which a plurality of pyrrole rings are covalently bonded; and an aliphatic resin having at least one kind of functional group selected from the group consisting of: a hydroxy group; and a carboxy group. The inventors of the present invention have presumed that the aliphatic resin improves the leakage resistance property, and an increase in series resistance of a film is suppressed by an electronic interaction between the cyclic conjugated compound and the aliphatic resin, and the suppression contributes to an improvement in conversion efficiency.

The inventors of the present invention have conceived that the aliphatic resin having at least one kind of functional group selected from the group consisting of: a hydroxy group; and a carboxy group can contribute to the improvements in leakage resistance property and conversion efficiency when combined with the cyclic conjugated compound in which the plurality of pyrrole rings are covalently bonded. This is conceived to be because, when the plurality of pyrrole rings are covalently bonded to form the cyclic conjugated compound, a π-electron cloud is largely extended in a direction perpendicular to a large planar cyclic surface, and thus an electronic interaction with another molecule becomes larger. Electronic bias is caused inside the cyclic conjugated compound by the electronic interaction of the cyclic conjugated compound, which easily interacts electronically, with the functional group of the aliphatic resin, and hence a carrier density is increased.

As a result, it is presumed that the conductivity of the cyclic conjugated compound itself, which is a charge-transporting material, increases, and the increase contributes to the improvements in leakage resistance property and conversion efficiency. Further, it has been found that the charge-transporting layer contributes to the improvements in leakage resistance property and conversion efficiency even when a hole-transporting layer or an insulating layer is introduced in a thickness of several tens of nanometers between the charge-transporting layer and photoelectric conversion layer of the present invention because a defect of the crystal having a perovskite structure reach several tens to several hundreds of nanometers. The charge-transporting layer is particularly preferably brought into contact with the photoelectric conversion layer from the viewpoint of leakage resistance property.

Preferred specific examples of the hole-transporting layer or the insulating layer that may be interposed between the charge-transporting layer and photoelectric conversion layer of the present invention include sodium chloride, sodium iodide, potassium iodide, rubidium iodide, cesium acetate, copper(I) bromide, copper(I) iodide, nickel(II) chloride, zinc iodide, germanium dioxide, aluminum acetylacetonate, europium(III) acetylacetonate, 1,8-diaminooctane dihydroiodide, 1,4-butanediamine dihydroiodide, hexylamine hydrobromide, n-octylamine hydrobromide, 2-phenylethylammonium iodide, ethylenediamine dihydroiodide, sodium fluoride, cesium chloride, methylammonium chloride, lead(II) thiocyanate, lead(II) acetate, potassium chloride, niobium(V) fluoride, choline chloride, L-α-phosphatidylcholine, fullerene, phenyl C61 butyric acid methyl ester (PCBM ((6,6)-phenyl C61 butyric acid methyl)), iodopentafluorobenzene, F4TCNQ, thiophene, pyridine, pentafluorobenzyl bromide, (3-mercaptopropyl)trimethoxysilane, thiourea, benzylamine, hexamethylenetetramine, N-(3-aminopropyl)-2-pyrrolidinone, theophylline, caffeine, 2-aminoethanesulfonamide hydrochloride, tri-n-octylphosphine oxide, graphene oxide, poly(3-hexylthiophene-2,5-diyl), poly(4-vinylpyridine), polyethylene oxide, polyvinylpyrrolidone, and poly(methyl methacrylate). Of those, the following are particularly preferred: sodium chloride, potassium iodide, rubidium iodide, cesium acetate, nickel(II) chloride, aluminum acetylacetonate, n-octylamine hydrobromide, 2-phenylethylammonium iodide, sodium fluoride, cesium chloride, methylammonium chloride, potassium chloride, niobium(V) fluoride, thiophene, pyridine, trimethoxysilane, thiourea, benzylamine, theophylline, poly(4-vinylpyridine), and poly(methyl methacrylate).

The molecular weight of the aliphatic resin is preferably 10,000 or more from the viewpoint of leakage resistance property.

In addition, the functional group of the aliphatic resin having at least one kind of functional group selected from the group consisting of: a hydroxy group; and a carboxy group preferably further includes at least one kind of functional group selected from the group consisting of: a carbonyl group; an ester group (ester bond); an ether group (ether bond); a carboxy group; a methoxy group; an amino group; a sulfo group; an aldehyde group; an amide group (amide bond); and a sulfide group from the viewpoint of an interaction with the cyclic conjugated compound.

The content of the cyclic conjugated compound in which the plurality of pyrrole rings are covalently bonded is preferably 5 to 30 mass%, more preferably 8 to 20 mass% with respect to the aliphatic resin having at least one kind of functional group selected from the group consisting of: a hydroxy group; and a carboxy group from the viewpoints of leakage resistance property and conversion efficiency.

The aliphatic resin having at least one kind of functional group selected from the group consisting of: a hydroxy group; and a carboxy group in the present invention refers to a resin having the functional group in any one of a main chain or a side chain. The functional group is preferably included at a position except an end of the main chain. In addition, a hydroxy group and a carboxy group are Lewis basic functional groups.

Preferred specific examples of the aliphatic resin to be preferably used in the present invention include polyvinyl alcohol, polyacrylic acid, poly(2-propylacrylic acid), poly(4-vinylphenol), polyvinyl butyral, poly(butadiene/maleic acid), poly(2-hydroxyethyl methacrylate), and poly(methyl methacrylate/methacrylic acid). Of those, polyvinyl alcohol, polyacrylic acid, polyvinyl butyral, poly(butadiene/maleic acid), poly(2-hydroxyethyl methacrylate), and poly(methyl methacrylate/methacrylic acid) are particularly preferred from the viewpoint of an electronic interaction.

The cyclic conjugated compound in which the plurality of pyrrole rings are covalently bonded to be used in the present invention is preferably a porphyrin compound or a phthalocyanine compound, more preferably a phthalocyanine compound from the viewpoint of the spread of a π-electron cloud that becomes a starting point of an interaction. The phthalocyanine compound may have a central element, and examples of the central element include Ga, Cu, Ti, Zn, Si, V, Pb, and Pt. Of those, Ga is preferred from the viewpoint of an electronic interaction with the aliphatic resin having at least one kind of functional group selected from the group consisting of: a hydroxy group; and a carboxy group, and a hydroxygallium phthalocyanine compound is particularly preferred from the viewpoint of an interaction with the functional group.

The aliphatic resin having at least one kind of functional group selected from the group consisting of: a hydroxy group; and a carboxy group in the present invention preferably has a glass transition temperature of 95°C or less from the viewpoint of a mixed state of a film.

Specific examples of the porphyrin compound of the present invention include the following compounds.

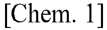

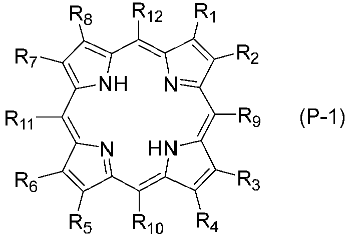

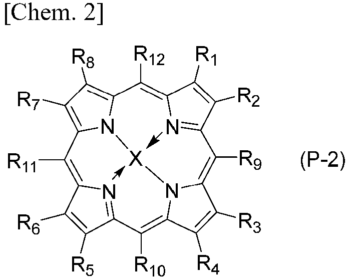

In the formulae (P-1) and (P-2), R₁ to R₁₂ each independently represent hydrogen, or an organic group including an aromatic group that may have a substituent or an aliphatic group that may have a substituent.

R₁ to R₁₂ each preferably represent hydrogen, a methyl group, an ethyl group, a propyl group, an isopropyl group, a butyl group, an octyloxy group, a butoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a sulfonic acid sodium salt group, a 4-cumylphenoxy group, a sulfonic acid group, a phenylthio group, a tert-butyl group, a hydroxy group, a carbonyl group, a methoxy group, an amino group, a sulfo group, or an aldehyde group. In the formulae (P-1) and (P-2), X represents an inorganic atom including a metal atom, and preferred specific examples thereof include Ga, GaOH, GaCl, TiO, Ti, Si, V, Pb, SiCl₂, Cu, Zn, Pd, Pb, Ni, Pt, Co, MnCl, FeCl, VO, and RuCO.

Specific examples of the phthalocyanine compound of the present invention include the following compounds.

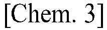

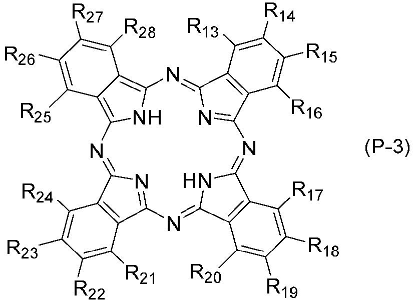

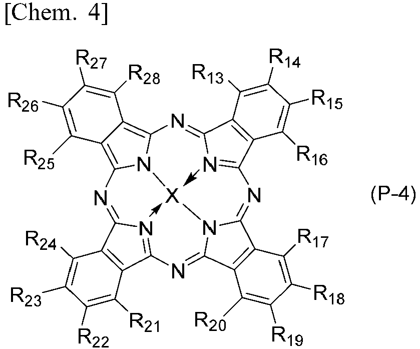

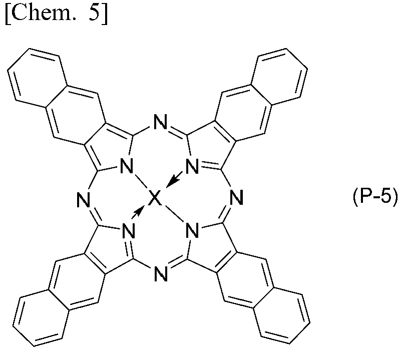

In the formulae (P-3) and (P-4), R₁₃ to R₂₈ each independently represent a hydrogen atom, or an organic group including an aromatic group that may have a substituent or an aliphatic group that may have a substituent. R₁₃ to R₂₈ each preferably represent a hydrogen atom, a methyl group, an ethyl group, a propyl group, an isopropyl group, a butyl group, an octyloxy group, a butoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a sulfonic acid sodium salt group, a 4-cumylphenoxy group, a sulfonic acid group, a phenylthio group, a tert-butyl group, a hydroxy group, a carbonyl group, a methoxy group, an amino group, a sulfo group, or an aldehyde group. Of those, the following are preferred: a methyl group, an ethyl group, a propyl group, a butyl group, a halogen atom, a sulfonic acid group, a hydroxy group, a carbonyl group, a methoxy group, an amino group, a sulfo group, and an aldehyde group.

In the formulae (P-3), (P-4), and (P-5), X represents an inorganic atom including a metal atom, and specific examples thereof include Ga, GaOH, GaCl, TiO, Ti, Si, V, Pb, SiCl₂, Cu, Zn, Pd, Pb, Ni, Pt, Co, MnCl, FeCl, VO, and RuCO. Of those, Ga, GaOH, GaCl, TiO, Ti, SiCl₂, Cu, Zn, Pd, Pb, Ni, Pt, Co, MnCl, FeCl, VO, and RuCO are preferred, and Ga, GaOH, GaCl, and TiO are more preferred.

As in the above-mentioned mechanism, when the respective constituent elements for forming the present invention exert synergistic effects on each other, the effect of the present invention can be achieved.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of an element to be incorporated gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and observing the element distribution of a specific element.

In addition, the chemical structure of a functional group or the like may be identified by a nuclear magnetic resonance method (NMR) and Fourier transform infrared spectroscopy (FT-IR).

Fig. 1 is a sectional view for schematically illustrating the configuration of a photoelectric conversion element according to one embodiment of the present invention. A photoelectric conversion element 1 of Fig. 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is an anode, and the other is a cathode. A current can be extracted by connecting the first electrode 7 and the second electrode 3 with an external circuit.

The photoelectric conversion layer 5 is excited by light that has entered the layer through the substrate 2, and the second electrode 3 and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes 3 and 7, and may not be formed in some cases. A form in which the plurality of electron-transporting layers 4 and photoelectric conversion layers 5 are laminated may be adopted. Such form may also be referred to as "tandem structure." In addition, the photoelectric conversion element may be produced in the order of the first electrode 7, the charge-transporting layer 6, the photoelectric conversion layer 5, the electron-transporting layer 4, and the second electrode 3 on the substrate 2.

Each member for forming the photoelectric conversion element of the present invention is described below.

### [Photoelectric Conversion Element]

The photoelectric conversion element of the present invention is a photoelectric conversion element including: the first electrode; the second electrode; and the photoelectric conversion layer arranged between the first electrode and the second electrode, the photoelectric conversion layer containing the crystal having a perovskite structure, and is characterized in that the photoelectric conversion element includes the charge-transporting layer between the photoelectric conversion layer and the first electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a crystal having a perovskite structure in its photoelectric conversion layer.

A method of forming each of the layers including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

A material for the first electrode 7 or the second electrode 3 is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The transparent electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

AₒBₚX_{q} [1]

In the general formula [1], A represents a cation, B represents a cation, and X represents an anion.

"o", "p", and "q" satisfy 0≤o≤10, 0≤p≤10, and 0≤q≤20, respectively, and A, B, and X may each be formed of a single material, or a combination of two or more kinds of materials. An additive may be added to the extent that the general formula is satisfied. The general formula generally forms a perovskite crystal having a three-dimensional structure, but when the cation A to be formed is large enough to fit within a crystal having a three-dimensional perovskite structure, a crystal having a two-dimensional perovskite structure, a crystal having a 2.5-dimensional perovskite structure having both the properties of two-dimensional and three-dimensional perovskite structures, a two-layer crystal having three-dimensional and two-dimensional perovskite structures, or a crystal having a mixed three-dimensional/two-dimensional perovskite structure is formed, and each of the crystals functions as a photoelectric conversion layer.

The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed. The crystal having a two-dimensional perovskite or 2.5-dimensional perovskite structure may form a perovskite structure of a Ruddlesden-Popper (RP) type, a Dion-Jacobson (DJ) type, or an alternating cations in the interlayer (ACI) type.

As A of the general formula [1], the cation A is not particularly limited. The cation A may or may not have a substituent, and specific examples thereof include the following structural formulae.

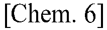

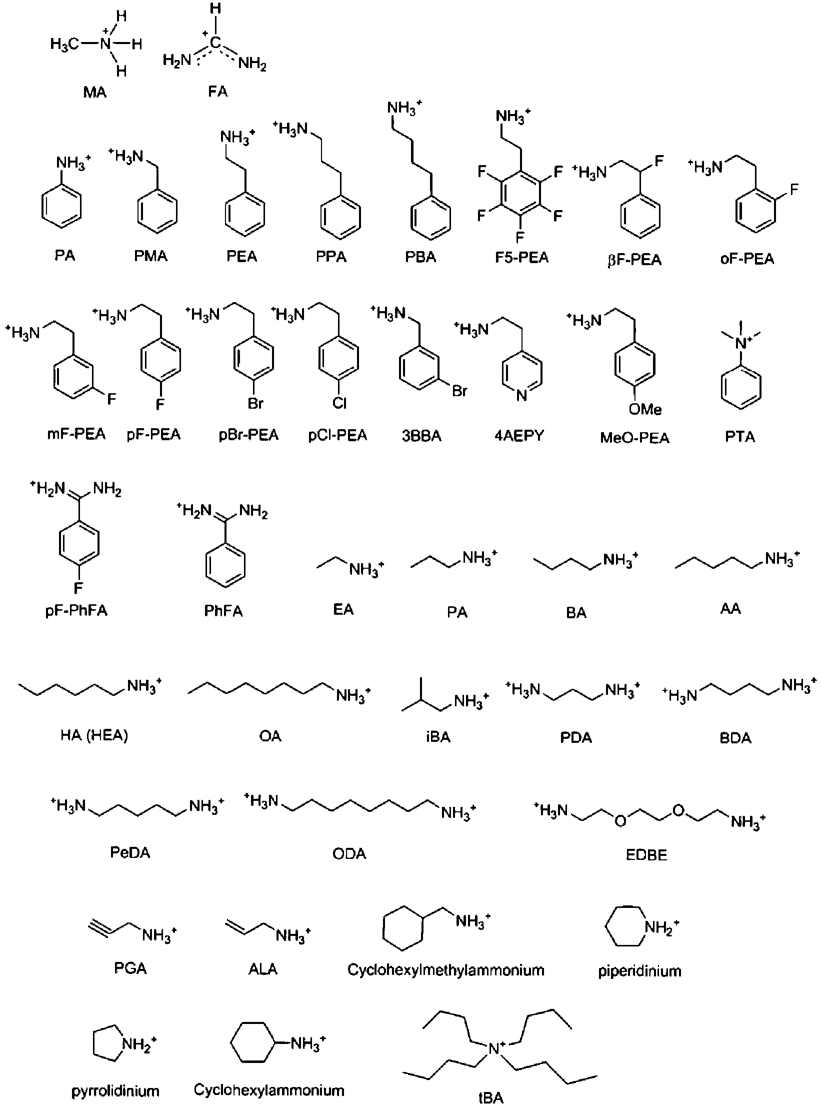

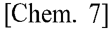

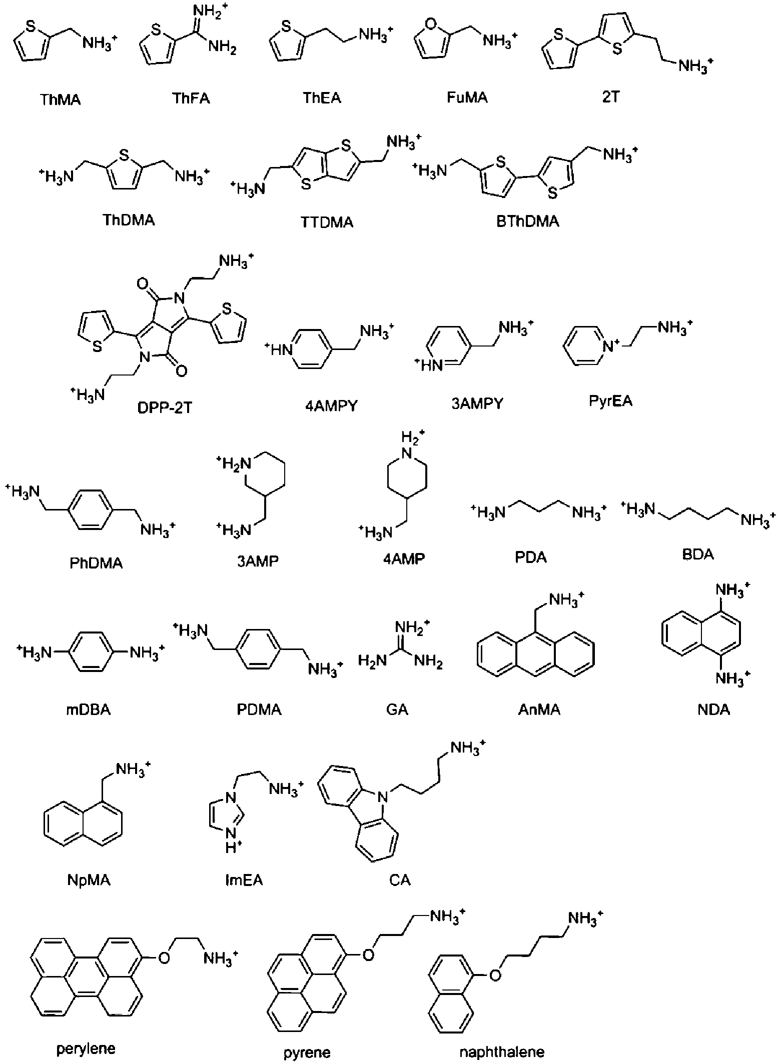

In addition, an inorganic atom is not particularly limited, and lithium, cesium, sodium, potassium, and rubidium are preferred. Those organic molecules or inorganic atoms may be used alone or in combination thereof.

B in the general formula [1] represents a cation atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, bismuth, and silver are preferred from the viewpoint of the stability of a perovskite crystal structure. Those atoms may be used alone or in combination thereof.

X in the general formula [1] represents a halogen or chalcogen atom, and examples thereof include chlorine, bromine, iodine, oxygen, sulfur, selenium, tellurium, and polonium. Those halogen or chalcogen atoms may be used alone or in combination thereof. Of those, a halogen atom is preferred because, when the halogen atom is incorporated into the structure, the above-mentioned crystal having a perovskite structure easily becomes soluble in an organic solvent, and hence its application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (pF-PEA)₂(FA_{0.3}MA_{0.7})₄Pb₅I₁₆, (PDMA)FA₂Pb₃I₁₀, (3AMPY)(MA)₃Pb₄I₁₃, (PDMA)A₅Pb₆I₁₉, (PDMA)MA₃Pb₄I₁₃, (BA_{0.9}PEA_{0.1})₂MA₄Pb₅I₁₆, (BA_{0.9}PEA_{0.1})₂MA₃Pb₄I₁₃, (BA)₂MA₂Pb₃I₁₀, (BA)₂MA₃Pb₄I₁₃, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred.

The A site, B site, or X site of each of the general formulae may be adjusted to be deficient or excessive in accordance with purposes, and the combinations of x1 to x5 may be changed in accordance with purposes. Examples of the combinations of x1 to x5 are as shown in Table 1. Particularly preferred ranges of the combinations of x1 to x5 are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

The crystal having a perovskite structure to be used in the present invention is preferably a crystalline semiconductor. The term "crystalline semiconductor" means a semiconductor that enables the measurement of an X-ray scattering intensity distribution to detect a scattering peak. When the crystal having a perovskite structure is the crystalline semiconductor, the mobility of an electron in the crystal having a perovskite structure increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm, a more preferred upper limit is 1,200 nm, a still more preferred lower limit is 100 nm, and a still more preferred upper limit is 1,000 nm.

### [Charge-transporting Layer]

In the present invention, the charge-transporting layer preferably contains a cyclic conjugated compound in which a plurality of pyrrole rings are bonded by conjugated bonds, and at least one kind of functional group selected from the group consisting of: a hydroxy group; and a carboxy group.

Further, the molecular weight of the aliphatic resin having at least one kind of functional group selected from the group consisting of: a hydroxy group; and a carboxy group is preferably 10,000 or more.

The aliphatic resin having at least one kind of functional group selected from the group consisting of: a hydroxy group; and a carboxy group preferably further has at least one kind of functional group selected from the group consisting of: a carbonyl group; an ester group; an ether group; a carboxy group; a methoxy group; an amino group; a sulfo group; an aldehyde group; an amide group; and a sulfide group.

The content mass of the cyclic conjugated compound in the charge-transporting layer is preferably a 5 to 30-fold amount, more preferably an 8 to 20-fold amount with respect to the content mass of the aliphatic resin in the charge-transporting layer.

The cyclic conjugated compound in the charge-transporting layer is preferably a phthalocyanine compound, and the phthalocyanine compound preferably has a central element. Further, it is preferred that the phthalocyanine compound be a metal phthalocyanine compound, preferably a gallium phthalocyanine compound, more preferably a hydroxygallium phthalocyanine compound.

The thickness of the charge-transporting layer is preferably 1 to 1,000 nm, more preferably 5 to 500 nm, particularly preferably 10 to 200 nm.

The charge-transporting layer may be formed by preparing a coating liquid for a charge-transporting layer containing the above-mentioned respective materials and a solvent, forming a coating film of the liquid on the photoelectric conversion layer, and drying the coating film. Examples of the solvent to be used for the coating liquid include an alcohol-based solvent, a ketone-based solvent, an etherbased solvent, an ester-based solvent, and an aromatic hydrocarbon-based solvent. Of those solvents, an alcohol-based solvent or an aromatic hydrocarbon-based solvent is preferred.

### [Second Charge-transporting Layer]

The photoelectric conversion element 1 in the present invention may further include a second charge-transporting layer between the charge-transporting layer 6 and the first electrode 7 from the viewpoint of the compatibility of a film of the charge-transporting layer 6.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. In particular, the second charge-transporting layer preferably has an aromatic ring from the viewpoint of the compatibility of a film interface, preferably contains a spirofluorene compound or a triphenylamine compound, and preferably contains Spiro-OMeTAD or PTAA.

In addition, the second charge-transporting layer may contain a dopant as an additive in order to improve its charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element of the present invention, the electron-transporting layer 4 may be arranged between the second electrode 3 and the photoelectric conversion layer 5 as illustrated in Fig. 1 and Fig. 2.

A material for the electron-transporting layer 4 is not particularly limited, and examples thereof include an N-type conductive polymer, an N-type lowmolecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene compound, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide.

A preferred lower limit of the thickness of the electron-transporting layer 4 is 1 nm, and a preferred upper limit thereof is 2,000 nm. When such thickness is 1 nm or more, a hole can be sufficiently blocked, and when the thickness is 2,000 nm or less, the electron-transporting layer 4 is less liable to serve as a resistance at the time of electron transportation, and hence the photoelectric conversion efficiency increases. A more preferred lower limit of the thickness is 3 nm, a more preferred upper limit thereof is 1,000 nm, a still more preferred lower limit thereof is 5 nm, and a still more preferred upper limit thereof is 500 nm.

### [Photoelectric Conversion Apparatus]

A photoelectric conversion apparatus of the present invention includes the photoelectric conversion element of the present invention. The photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." In the photoelectric conversion element, elements having different absorption wavelengths may be laminated to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function of, for example, maintaining or increasing the amount of incident light, a surface layer to which water or dirt is hard to adhere, or a function of collecting or guiding light may be added.

### [Moving Body]

Fig. 3 is a perspective view for schematically illustrating one embodiment of a moving body including the photoelectric conversion element of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

Fig. 4 is a perspective view for schematically illustrating one embodiment of a building material including the photoelectric conversion element of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. In general, when the building material including the photoelectric conversion element is used for a roof or the like, the temperature of the photoelectric conversion element 41 may be increased by sunlight, and hence the photoelectric conversion efficiency may be reduced. In this case, the reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exteriors 44a and 44b may be formed of the same member, or may be formed of different members. A paint or a transparent substrate may be used as an exterior member. An exterior having small light absorption and a high heat-shielding property is preferably used.

In addition to the application examples described above, the following application examples of the photoelectric conversion element of the present invention may be given: portable devices, such as a calculator, a sensor, and a small solar panel; wearable devices, such as a glasses-type terminal, a watch-type terminal, and a portable medical device; sheet structures supported by a plurality of frames, such as a tent, a plastic house, and a loading platform of a truck; and structures to be used by being fixed, such as a road surface panel, a floating panel, a building material utilizing the flexibility of a substrate, a wall-type building material, a glass-type building material, and a mega solar panel.

### [Method of producing Photoelectric Conversion Element]

A method of producing the photoelectric conversion element of the present invention includes the steps of: forming a first electrode; forming a second electrode; forming a photoelectric conversion layer containing a crystal having a perovskite structure between the first electrode and the second electrode; and forming a charge-transporting layer between the photoelectric conversion layer and the first electrode.

The respective steps of the production method are described below.

### [Step of forming First Electrode and Step of forming Second Electrode]

In the step of forming the first electrode and the step of forming the second electrode, appropriate methods may be selected in accordance with a material of the first electrode and a material of the second electrode, respectively. Examples of such methods include, but are not limited to, a sputtering method, a vacuum vapor deposition method, a vapor phase growth method (CVD method), and a spray pyrolysis deposition method (SPD method). Materials of the first electrode and the second electrode are as described above. When one, or each of both, of the first electrode and the second electrode is a transparent electrode, the thickness of the transparent electrode is preferably 0.03 to 3 µm.

When a solar cell is produced, cutting processing may be performed for circuit formation between steps. Examples of the cutting processing include mechanical patterning and laser patterning.

### [Modularization Step]

An element formed up to the electrode may be sealed. A sealing method is, for example, sealing with a resin or sealing with a film. Examples of a material used for the sealing include silazane, silicone rubber, resins each having a siloxane skeleton, and glass.

In addition, hairline treatment may be applied to the surface of the sealed element from the viewpoint of the suppression of adhesion between elements occurring during winding in a roll to roll system.

### [Step of forming Photoelectric Conversion Layer]

The step of forming the photoelectric conversion layer may include a step of applying a liquid containing the material of the photoelectric conversion layer as described above. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with the properties of a photoelectric conversion layer to be produced, such as thickness control and orientation control. Annealing treatment may be performed under reduced pressure or in an inert atmosphere (in a nitrogen or argon atmosphere) in order to remove a solvent or a dispersion medium from the applied liquid containing the material of the photoelectric conversion layer. The temperature of the annealing treatment is preferably 40 to 300°C, more preferably 50 to 150°C. The annealing treatment is preferably performed because materials for forming the respective layers permeate each other at an interface between laminated layers to increase a contact area, and hence a short-circuit current can be increased in some cases.

### [Step of forming Charge-transporting Layer]

As the step of forming the charge-transporting layer, a method of applying a liquid containing the material of the charge-transporting layer as described above is preferred. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method.

### Examples

The present invention is described in more detail below by way of Examples and Comparative Examples. The present invention is by no means limited to the following Examples without departing from the gist thereof. In the description of the following Examples, the term "part(s)" is by mass unless otherwise specified.

### <Step of producing Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (OHGaPc) particle (crystal) having a water content of 1.0 mass% or less was obtained.

### Step (3)

5 Parts of the hydroxygallium phthalocyanine particle was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) containing 5 parts of glass beads, followed by filtration and drying to provide a particle 1.

### <Production of Resin Solution 1>

1.0 Gram of polyvinyl butyral (product name: BM-2, manufactured by Sekisui Chemical Co., Ltd., glass transition temperature: 71°C) was dissolved in 19 g of 2-propanol by stirring for 24 hours to provide a resin solution 1.

### (Example 1)

### [Formation of Electron-transporting Layer]

A glass substrate with ITO was washed, and tin(II) oxide whose concentration had been adjusted to 3 mass% was applied thereonto by spin coating. After that, the resultant was heated at 150°C for 30 minutes to form an electron-transporting layer as a thin film having a thickness of 15 nm.

### [Formation of Photoelectric Conversion Layer]

22.4 Milligrams of methylammonium bromide, 172 mg of formamidinium iodide, and 576 mg of lead iodide were dissolved in 600 µL of N,N-dimethylformamide and 160 µL of dimethyl sulfoxide, and the solution was stirred for 1 hour (solution 1). Further, 389.72 mg of cesium iodide was dissolved in 1,000 µL of dimethyl sulfoxide, and the solution was stirred for 1 hour (solution 2). After that, 40 µL of the cesium iodide solution (solution 2) was added to the solution 1 to prepare a coating liquid for a photoelectric conversion layer. The coating liquid was applied onto the electron-transporting layer by spin coating to form a photoelectric conversion layer formed of Cs_{0.05}(FA_{0.83}MA_{0.17})_{0.96}Pb(I_{0.95}Br_{0.05})₃ and having a thickness of 500 nm.

### [Formation of Charge-transporting Layer]

0.1 Gram of the particle 1 and 0.01 g of a calixarene compound (Japanese Patent Laid-Open No. 2003-207913) were mixed with 10.6 g of 2-propanol, and 11 g of zirconia beads were loaded into the mixture, followed by paint shaker dispersion (manufactured by Toyo Seiki Co., Ltd.) for 6 hours. After that, 0.2 g of the resin solution 1 was added thereto, and paint shaker dispersion was performed again for 6 hours to prepare a coating liquid for a charge-transporting layer. The coating liquid for a charge-transporting layer was applied onto the photoelectric conversion layer by spin coating to form a charge-transporting layer having a thickness of 150 nm.

### [Formation of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithium bis(trifluoromethanesulfonyl)imide in 0.3 g of acetonitrile and 60 µL of 4-tert-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a material solution for a second charge-transporting layer. The solution was applied onto the above-mentioned charge-transporting layer by a spin coating method to form a second charge-transporting layer having a thickness of 200 nm.

### [Formation of First Electrode]

A gold electrode having a thickness of 80 nm and an area of 0.09 cm² was formed on the second charge-transporting layer by a vacuum vapor deposition method. Thus, a photoelectric conversion element was obtained.

### [Analysis of Amount of Compound]

The electrode surface of the photoelectric conversion element was peeled off to expose the surface of the charge-transporting layer. The surface of the charge-transporting layer was wiped with a cotton swab or the like with a solvent, dissolved in deuterated sulfuric acid, and subjected to ¹H-NMR measurement (apparatus: AVANCE 3-500 manufactured by BRUKER). In addition, the mass and structure analysis of the peeled-off charge-transporting layer components was performed by elemental analysis, such as GPC and MALDI-TOF-MS, IR, gas chromatography, XPS, and EDX, to recognize the presence of a compound.

In addition, the thickness of the charge-transporting layer was observed with a cross-sectional SEM (apparatus: SmartSEM manufactured by Carl Zeiss Co., Ltd.) after the cutting of the photoelectric conversion element and the fixing of the sample to a tilted sample stage. With regard to the crystallinity of each of the materials, diffraction peaks were observed by XRD measurement (apparatus: X-ray diffractometer RINT-TTRII manufactured by Rigaku Corporation).

### (Example 2)

A photoelectric conversion element is obtained in the same manner as in Example 1 except that a polyvinyl butyral resin (product name: BM-S, manufactured by Sekisui Chemical Co., Ltd., glass transition temperature: 67°C) having a different ratio of hydroxy groups is used as the aliphatic resin having a hydroxy group.

### (Example 3)

A thin film layer having a thickness of 20 nm is formed between the charge-transporting layer and the photoelectric conversion layer by: dissolving 2.49 mg of 2-phenylethylamine hydroiodide into 1 mL of 2-propanol to prepare a solution for a thin film layer; and applying the solution for a thin film layer onto the photoelectric conversion layer by spin coating. A photoelectric conversion element is obtained in the same manner as in Example 1 except the foregoing.

### (Example 4)

The cyclic conjugated compound in which the plurality of pyrrole rings are bonded by conjugated bonds is changed to chlorogallium phthalocyanine (ClGaPc). A photoelectric conversion element is obtained in the same manner as in Example 1 except the foregoing.

### (Example 5)

The cyclic conjugated compound in which the plurality of pyrrole rings are bonded by conjugated bonds is changed to copper phthalocyanine (CuPc). A photoelectric conversion element is obtained in the same manner as in Example 1 except the foregoing.

### (Example 6)

The cyclic conjugated compound in which the plurality of pyrrole rings are bonded by conjugated bonds is changed to titanyl phthalocyanine (TiOPc). A photoelectric conversion element is obtained in the same manner as in Example 1 except the foregoing.

### (Example 7)

The cyclic conjugated compound in which the plurality of pyrrole rings are bonded by conjugated bonds is changed to zinc phthalocyanine (ZnPc). A photoelectric conversion element is obtained in the same manner as in Example 1 except the foregoing.

### (Example 8)

The cyclic conjugated compound in which the plurality of pyrrole rings are bonded by conjugated bonds is changed to silicon phthalocyanine dichloride (SiPcCl₂). A photoelectric conversion element is obtained in the same manner as in Example 1 except the foregoing.

### (Example 9)

The cyclic conjugated compound in which the plurality of pyrrole rings are bonded by conjugated bonds is changed to ligand-free phthalocyanine (Pc). A photoelectric conversion element is obtained in the same manner as in Example 1 except the foregoing.

### (Example 10)

The cyclic conjugated compound in which the plurality of pyrrole rings are bonded by conjugated bonds is changed to tetraphenylporphyrin (TPP). A photoelectric conversion element is obtained in the same manner as in Example 1 except the foregoing.

### (Example 11)

A photoelectric conversion element is obtained in the same manner as in Example 1 except that 0.2 g of the particle 1 is used for the preparation of the coating liquid 1 for a charge-transporting layer.

### (Example 12)

A photoelectric conversion element is obtained in the same manner as in Example 1 except that 0.08 g of the particle 1 is used for the preparation of the coating liquid 1 for a charge-transporting layer.

### (Example 13)

A photoelectric conversion element is obtained in the same manner as in Example 1 except that 0.3 g of the particle 1 is used for the preparation of the coating liquid 1 for a charge-transporting layer.

### (Example 14)

A photoelectric conversion element is obtained in the same manner as in Example 1 except that 0.05 g of the particle 1 is used for the preparation of the coating liquid 1 for a charge-transporting layer.

### (Example 15)

A photoelectric conversion element is obtained in the same manner as in Example 1 except that 0.35 g of the particle 1 is used for the preparation of the coating liquid 1 for a charge-transporting layer.

### (Example 16)

A photoelectric conversion element is obtained in the same manner as in Example 1 except that 0.02 g of the particle 1 is used for the preparation of the coating liquid 1 for a charge-transporting layer.

### (Example 17)

In the production of the resin solution 1, the aliphatic resin having a hydroxy group is changed to KS-10 (manufactured by Sekisui Chemical Co., Ltd., glass transition temperature: 105°C). A photoelectric conversion element is obtained in the same manner as in Example 1 except the foregoing.

### (Example 18)

In the production of the resin solution 1, the resin is changed to PBMA (compound represented by the following formula (E-1)), which is an aliphatic resin having a carboxy group. A photoelectric conversion element is obtained in the same manner as in Example 1 except the foregoing.

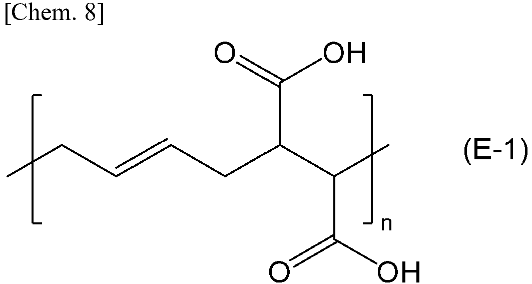

### (Example 19)

In the production of the resin solution 1, the resin is changed to PHM (compound represented by the following formula (E-2)), which is an aliphatic resin having a hydroxy group. A photoelectric conversion element is obtained in the same manner as in Example 1 except the foregoing.

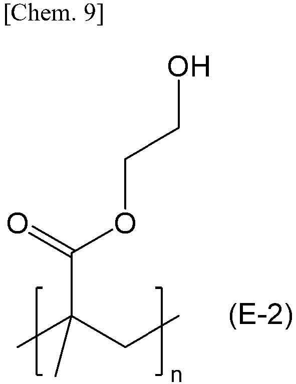

### (Example 20)

In the production of the resin solution 1, the resin is changed to PVA (compound represented by the following formula (E-3)), which is an aliphatic resin having a hydroxy group. A photoelectric conversion element is obtained in the same manner as in Example 1 except the foregoing.

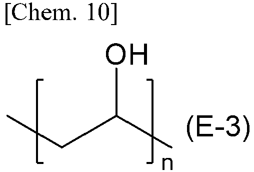

### (Example 21)

In the production of the resin solution 1, the resin is changed to PMMMA (compound represented by the following formula (E-4)), which is an aliphatic resin having a hydroxy group. A photoelectric conversion element is obtained in the same manner as in Example 1 except the foregoing.

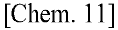

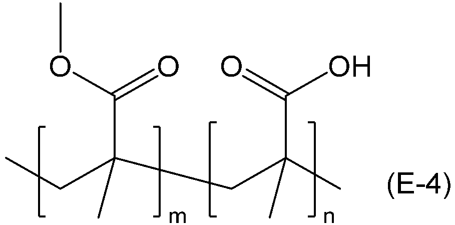

### (Example 22)

A photoelectric conversion element is obtained in the same manner as in Example 1 except that the second charge-transporting layer is not used.

### (Example 23)

In the production of the resin solution 1, the aliphatic resin having a hydroxy group is changed to BX-1 (manufactured by Sekisui Chemical Co., Ltd., glass transition temperature: 95°C). A photoelectric conversion element is obtained in the same manner as in Example 1 except the foregoing.

### (Example 24)

In the production of the resin solution 1, the resin is changed to polyacrylic acid (weight-average molecular weight: 25,000, manufactured by FUJIFILM Wako Pure Chemical Corporation), which is an aliphatic resin having a carboxy group, and 2-propanol is changed to ethanol. A photoelectric conversion element is obtained in the same manner as in Example 1 except the foregoing.

### (Comparative Example 1)

A photoelectric conversion element was obtained in the same manner as in Example 1 except that, in the production of the resin solution 1, the aliphatic resin having at least one kind of group selected from the group consisting of: a hydroxy group; and a carboxy group was not used (i.e., the resin solution 1 was not used).

### (Comparative Example 2)

A photoelectric conversion element is obtained in the same manner as in Example 1 except that, in the production of the coating liquid for a charge-transporting layer, the cyclic conjugated compound in which the plurality of pyrrole rings are bonded by conjugated bonds is not used.

### (Comparative Example 3)

A photoelectric conversion element is obtained in the same manner as in Example 1 except that, in the production of the coating liquid for a charge-transporting layer, the cyclic conjugated compound in which the plurality of pyrrole rings are bonded by conjugated bonds is not used and is changed to Spiro-OMeTAD instead.

### (Comparative Example 4)

A photoelectric conversion element is obtained in the same manner as in Example 1 except that, in the production of the resin solution 1, the aliphatic resin having at least one kind of group selected from the group consisting of: a hydroxy group; and a carboxy group is not used, and is changed to poly(3-hexylthiophene-2,5-diyl) P3HT (manufactured by Sigma-Aldrich CO. LLC, glass transition temperature: 9.3°C) instead.

### (Comparative Example 5)

In the production of the resin solution 1, the aliphatic resin having at least one kind of group selected from the group consisting of: a hydroxy group; and a carboxy group is changed to KUREHA KF Polymer (manufactured by Kuraray Co., Ltd., glass transition temperature: -35°C). A photoelectric conversion element is obtained in the same manner as in Example 1 except the foregoing.

### (Comparative Example 6)

A photoelectric conversion element is obtained in the same manner as in Example 1 except that: in the production of the resin solution 1, the aliphatic resin having at least one kind of group selected from the group consisting of: a hydroxy group; and a carboxy group is changed to PSTFSI (compound represented by the following formula (E-5)); and the cyclic conjugated compound in which the plurality of pyrrole rings are bonded by conjugated bonds is changed to copper phthalocyanine (CuPc).

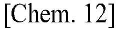

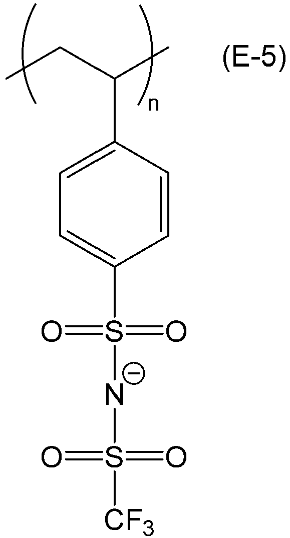

### (Comparative Example 7)

A photoelectric conversion element is obtained in the same manner as in Example 1 except that, in the production of the resin solution 1, the aliphatic resin having at least one kind of group selected from the group consisting of: a hydroxy group; and a carboxy group is changed to PSTFSI.

The specific configuration of the charge-transporting layer, and the presence or absence of the second charge-transporting layer or the thin film layer in the photoelectric conversion element produced in each of Examples 1 to 24 and Comparative Examples 1 to 7 are shown in Table 2.

### [Evaluation]

With regard to the photoelectric conversion element obtained in each of Examples and Comparative Examples, the following evaluation was performed.

### (Power generation Efficiency Evaluation)

In Example 1, a power source (manufactured by Keithley Instruments, Model 236) was connected between the electrodes of the photoelectric conversion element, and constant light was applied with a solar simulator (manufactured by Yamashita Denso Corporation) at an intensity of 100 mW/cm², followed by the measurement of a current and a voltage to be generated. Thus, photoelectric conversion efficiency was evaluated. A series resistance was calculated by approximating the reciprocal of a slope near the Voc of the resultant current-voltage curve, and a shunt resistance was calculated by approximating the reciprocal of a slope near the Jsc of the resultant current-voltage curve. The photoelectric conversion element was evaluated for its leakage resistance property by the value of the series resistance and the value of the shunt resistance. Examples 2 to 24 and Comparative Examples 1 to 7 are each evaluated in the same manner as in Example 1 so that its photoelectric conversion efficiency may be determined. The results are shown as relative values when the result of Example 1 is set to 1. The results are shown in Table 3.

**[Table 2]**

| Example | Charge-transporting layer | | | | | | Second chargetransport ing layer | Thin film layer |
|---|---|---|---|---|---|---|---|---|
| | Cyclic conjugated compound | Functional group of resin in charge-transporting layer | | | Glass transition temperature [°C] | Content mass of cyclic conjugated compound relative to resin | | |
| Example 1 | OHGaPc | Ester group | Hydroxy group | Ether group | 71°C | 10-fold amount | Present | Absent |
| Example 2 | OHGaPc | Ester group | Hydroxy group | Ether group | 67°C | 10-fold amount | Present | Absent |
| Example 3 | OHGaPc | Ester group | Hydroxy group | Ether group | 71°C | 10-fold amount | Present | PEAI |
| Example 4 | ClGaPc | Ester group | Hydroxy group | Ether group | 71°C | 10-fold amount | Present | Absent |
| Example 5 | CuPc | Ester group | Hydroxy group | Ether group | 71°C | 10-fold amount | Present | Absent |
| Example 6 | TiOPc | Ester group | Hydroxy group | Ether group | 71°C | 10-fold amount | Present | Absent |
| Example 7 | ZnPc | Ester group | Hydroxy group | Ether group | 71°C | 10-fold amount | Present | Absent |
| Example 8 | SiPcCl₂ | Ester group | Hydroxy group | Ether group | 71°C | 10-fold amount | Present | Absent |
| Example 9 | Pc | Ester group | Hydroxy group | Ether group | 71°C | 10-fold amount | Present | Absent |
| Example 10 | TPP | Ester group | Hydroxy group | Ether group | 71°C | 10-fold amount | Present | Absent |
| Example 11 | OHGaPc | Ester group | Hydroxy group | Ether group | 71°C | 20-fold amount | Present | Absent |
| Example 12 | OHGaPc | Ester group | Hydroxy group | Ether group | 71°C | 8-fold amount | Present | Absent |
| Example 13 | OHGaPc | Ester group | Hydroxy group | Ether group | 71°C | 30-fold amount | Present | Absent |
| Example 14 | OHGaPc | Ester group | Hydroxy group | Ether group | 71°C | 5-fold amount | Present | Absent |
| Example 15 | OHGaPc | Ester group | Hydroxy group | Ether group | 71°C | 35-fold amount | Present | Absent |
| Example 16 | OHGaPc | Ester group | Hydroxy group | Ether group | 71°C | 2-fold amount | Present | Absent |
| Example 17 | OHGaPc | Ester group | Hydroxy group | Ether group | 105°C | 10-fold amount | Present | Absent |
| Example 18 | OHGaPc | Carboxy group | | | 57°C | 10-fold amount | Present | Absent |
| Example 19 | OHGaPc | Hydroxy group | Ester group | | 55°C | 10-fold amount | Present | Absent |
| Example 20 | OHGaPc | Hydroxy group | | | 85°C | 10-fold amount | Present | Absent |
| Example 21 | OHGaPc | Carboxy group | Ester group | | | 10-fold amount | Present | Absent |
| Example 22 | OHGaPc | Ester group | Hydroxy group | Ether group | 71°C | 10-fold amount | Absent | Absent |
| Example 23 | OHGaPc | Ester group | Hydroxy group | Ether group | 95°C | 10-fold amount | Present | Absent |
| Example 24 | OHGaPc | Carboxy group | | | | 10-fold amount | Present | Absent |
| Comparative Example 1 | OHGaPc | | | | | | Present | Absent |
| Comparative Example 2 | Absent | Ester group | Hydroxy group | Ether group | 71°C | | Present | Absent |
| Comparative Example 3 | Spiro-OMeTAD | Ester group | Hydroxy group | Ether group | 71°C | 10-fold amount | Present | Absent |
| Comparative Example 4 | OHGaPc | Thiophene | | | 9.3°C | 10-fold amount | Present | Absent |
| Comparative Example 5 | OHGaPc | Fluorine | | | -35°C | 10-fold amount | Present | Absent |
| Comparative Example 6 | CuPc | Sulfonyl group | | | 30°C | 10-fold amount | Present | Absent |
| Comparative Example 7 | OHGaPc | Sulfonyl group | | | 30°C | 10-fold amount | Present | Absent |

**[Table 3]**

| Example | Element characteristics | | |
|---|---|---|---|
| | Conversion efficiency relative value relative to Example 1 | Series resistance [Ω] | Shunt resistance [Ω] |
| Example 1 | 1.00 | 1.00 | 1.00 |
| Example 2 | 0.99 | 1.12 | 0.99 |
| Example 3 | 0.97 | 1.21 | 1.02 |
| Example 4 | 0.94 | 1.30 | 0.97 |
| Example 5 | 0.91 | 1.49 | 0.93 |
| Example 6 | 0.91 | 1.60 | 0.95 |
| Example 7 | 0.90 | 1.56 | 0.96 |
| Example 8 | 0.87 | 1.77 | 0.91 |
| Example 9 | 0.85 | 2.04 | 0.91 |
| Example 10 | 0.84 | 2.27 | 0.90 |
| Example 11 | 0.98 | 0.95 | 0.93 |
| Example 12 | 0.97 | 1.35 | 1.07 |
| Example 13 | 0.93 | 0.92 | 0.87 |
| Example 14 | 0.91 | 2.11 | 1.27 |
| Example 15 | 0.89 | 0.87 | 0.79 |
| Example 16 | 0.46 | 23.2 | 2.40 |
| Example 17 | 0.88 | 2.47 | 0.93 |
| Example 18 | 0.92 | 1.29 | 0.96 |
| Example 19 | 0.95 | 1.24 | 0.93 |
| Example 20 | 0.91 | 1.32 | 0.96 |
| Example 21 | 0.93 | 1.29 | 0.98 |
| Example 22 | 0.79 | 0.91 | 0.73 |
| Example 23 | 0.99 | 1.03 | 0.98 |
| Example 24 | 0.89 | 1.43 | 0.93 |
| Comparative Example 1 | 0.80 | 0.84 | 0.75 |
| Comparative Example 2 | 0.41 | 75.5 | 3.42 |
| Comparative Example 3 | 0.72 | 2.87 | 0.84 |
| Comparative Example 4 | 0.78 | 0.85 | 0.72 |
| Comparative Example 5 | 0.68 | 1.96 | 1.05 |
| Comparative Example 6 | 0.69 | 2.36 | 1.41 |
| Comparative Example 7 | 0.71 | 2.12 | 1.46 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-086015 filed on May 28, 2024, and Japanese Patent Application No. 2024-186450 filed on October 23, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises, between the photoelectric conversion layer and the first electrode, a charge-transporting layer containing:
a cyclic conjugated compound in which a plurality of pyrrole rings are bonded by conjugated bonds; and
an aliphatic resin having at least one kind of functional group selected from the group consisting of: a hydroxy group; and a carboxy group.

2. The photoelectric conversion element according to claim 1, wherein the photoelectric conversion element comprises a second charge-transporting layer between the first electrode and the charge-transporting layer.

3. The photoelectric conversion element according to claim 2, wherein the second charge-transporting layer contains a spirofluorene compound.

4. The photoelectric conversion element according to any one of claims 1 to 3, wherein the aliphatic resin further has at least one kind of functional group selected from the group consisting of: a carbonyl group; an ester group; an ether group; a carboxy group; a methoxy group; an amino group; a sulfo group; an aldehyde group; an amide group; and a sulfide group.

5. The photoelectric conversion element according to any one of claims 1 to 4, wherein a content mass of the cyclic conjugated compound in the charge-transporting layer is a 5 to 30-fold amount with respect to a content mass of the aliphatic resin in the charge-transporting layer.

6. The photoelectric conversion element according to any one of claims 1 to 5, wherein a content mass of the cyclic conjugated compound in the charge-transporting layer is an 8 to 20-fold amount with respect to a content mass of the aliphatic resin in the charge-transporting layer.

7. The photoelectric conversion element according to any one of claims 1 to 6, wherein the cyclic conjugated compound is a phthalocyanine compound.

8. The photoelectric conversion element according to claim 7, wherein the phthalocyanine compound has a central element.

9. The photoelectric conversion element according to claim 7 or 8, wherein the phthalocyanine compound is a metal phthalocyanine compound.

10. The photoelectric conversion element according to claim 9, wherein the metal phthalocyanine compound is a gallium phthalocyanine compound.

11. The photoelectric conversion element according to claim 10, wherein the gallium phthalocyanine compound is a hydroxygallium phthalocyanine compound.

12. The photoelectric conversion element according to any one of claims 1 to 11, wherein the aliphatic resin has a glass transition temperature of 95°C or less.

13. A photoelectric conversion apparatus comprising the photoelectric conversion element of any one of claims 1 to 12.
